Europäisches Patentamt

European Patent Office

Office européen des brevets

(19)

(11) Publication number: **0 250 192**
**A2**

## (12) EUROPEAN PATENT APPLICATION

(21) Application number: **87305286.4**

(22) Date of filing: **15.06.87**

(51) Int. Cl.⁴: **A61K 37/02 , A61K 45/05**

(30) Priority: **16.06.86 US 874736**
**22.05.87 US 53938**

(43) Date of publication of application:
**23.12.87 Bulletin 87/52**

(84) Designated Contracting States:
**AT BE CH DE ES FR GB GR IT LI LU NL SE**

(71) Applicant: **GENENTECH, INC.**
**460 Point San Bruno Boulevard**
**South San Francisco California 94080(US)**

(72) Inventor: **Palladino, Michael Angelo**
**511 Hanbury Lane**
**Foster City California 94404(US)**
Inventor: **Ammann, Arthur J**
**104 Dominican Drive**
**San Rafael California 94901(US)**

(74) Representative: **Armitage, Ian Michael et al**
**MEWBURN ELLIS & CO. 2/3 Cursitor Street**
**London EC4A 1BQ(GB)**

(54) **Therapeutic compositions.**

(57) Tumor necrosis factor-alpha is employed in the manufacture of a medicament for the treatment of infectious disease. Resultant formulations are particularly suitable for those infections consequent to a defect in phagocytic cells.

EP 0 250 192 A2

## THERAPEUTIC COMPOSITIONS

The present invention relates to the treatment of bacterial and fungal infections. In particular, the present invention relates to the use of cytokines in such treatment. Specifically the invention relates to the use of the cytokine, tumor necrosis factor-alpha (TNF-α) as an anti-infective agent.

The infections treated by the anti-infective agents of the instant invention are those occurring consequent to a defect in phagocytic cells. The phagocytes including monocytes, polymorphonuclear neutrophils, macrophages and eosinophils normally engulf bacterial and fungal pathogens. The phagocyte kills the engulfed microbe by an unknown mechanism which may utilize oxygen as evidenced by a "respiratory burst". In certain diseases the phagocytes are able to remove pathogens from the blood and tissues by engulfing them but fail to destroy the phagocytized microorganisms. These non-killing phagocytes harbor viable microorganisms. Chronic granulomatous disease (CGD) is an example of a condition rsulting from a genetic defect giving rise to non-killing polymorphonuclear neutrophils. Chronic granulomatous disease occurs most frequently in children but is also observed in adults. Patients with chronic granulomatous disease manifest an undue susceptibility to infections. Hakansson, L. et al., Arch. Dis. Child, 55 , 776 (1980). Chronic granulomatous disease constitutes a significant pediatric problem and is but one example of such a disease of defective phagocytes. The hallmark of CGD is essentially a complete absence of the respiratory burst by phagocytes that appear to be normal in all other respects. CGD patients have frequent severe and often fatal infections in their lymph nodes, liver, bone, skin and respiratory tract.

The respiratory burst evidences production of oxygen radicals. This oxygen consumption is believed to be required to produce optimum conditions for the killing of most common bacteria and fungal pathogens. Cells deprived of oxygen engulf but do not kill some microbes efficiently. Mandell, G.L., Infect. Immun. 9 , 337, 1974. The substances which may be associated with the killing of the pathogen and which require oxygen include superoxide, the hydroxyl radical, singlet oxygen and hydrogen peroxide.

A substance called tumor necrosis factor (TNF) was first described when patients undergoing spontaneous cancer regression were observed to have concurrent bacterial infections. Tumor necrosis factor-alpha appears to belong to a family of proteins which have varying degrees of similarity in biological activity and structure. Tumor necrosis factor-beta (formerly called lymphotoxin) also sometimes called cachectin is an example of another presently known molecule included in this family. While there has been speculation as to a possible role for tumor necrosis factor-alpha in infectious disease, tests on a range of bacteria and fungi have shown no evidence for direct tumor necrosis factor-alpha sensitivity. Reviewed in Old, L.J., Science 230, 632 (1985). Tumor necrosis factor-alpha has been shown to enhance the adherence of human neutrophils to human umbilical vein endothelial cell monolayers. Gamble, J.R. et al., PNAS 82, 8667 (1985). This enhancement of PMN adherence to endothelial cells was considered an element of tumor necrosis factor's effect in tumor rejection. Supra at 8667. Recombinant human interferon-gamma (rHuIFN-γ) and natural human tumor necrosis factor-beta (nHuTNF-β) caused an increase in vitro of PMN phagocytic ability as measured by PMN's ability to engulf latex beads. Shalaby, M.R., et al., ASBC/AAI (1984). Human interferon-gamma and human tumor necrosis factor-beta have been shown to enhance PMN-mediated antibody-dependent cellular cytotoxicity and also increase $0\overline{2}$ production. Shalaby, M.R. et al., J. of Immunology, 135 (3), 2069 (1985). Tumor necrosis factor has been shown to enhance human eosinophil killing of parasites in a dose-dependent fashion. Silberstein, D.S. and David, J.R., P.N.A.S. 83, 1055 (1986).

The present invention is based on the novel observation that human tumor necrosis factor-alpha activates polymorphonuclear neutrophils (PMN). Human tumor necrosis factor-alpha was found to enhance in vitro PMN's phagocytic ability. Human tumor necrosis factor-alpha was further found to reverse in vitro the inability of PMNs from male patients with X-linked CGD to generate a respiratory burst.

An object of the present invention is to provide an anti-infective therapeutic agent for various infectious diseases requiring phagocytic action. More specifically, human tumor necrosis factor-alpha may be used as an anti-infective agent in patients, having normal phagocytes, with various infections.

A further object of this inventin is to provide an anti-infective therapeutic agent to activate defective phagocytic cells.

The present invention is based on the novel observation that human tumor necrosis factor-alpha activates normal and defective polymorphonuclear neutrophils (PMN). Accordingly, in one aspect the invention is directed to the use of tumor necrosis factor-alpha in the manufacture of a medicament for treatment of an infectious disease. Thus, human tumor necrosis factor-alpha is used as an anti-infective therapy in infected patients wih normal phagocytes. In another aspect tumor necrosis factor is utilized in the manufacture of a composition for administration to patients with defective phagocytes to effectuate an antimicrobial response.

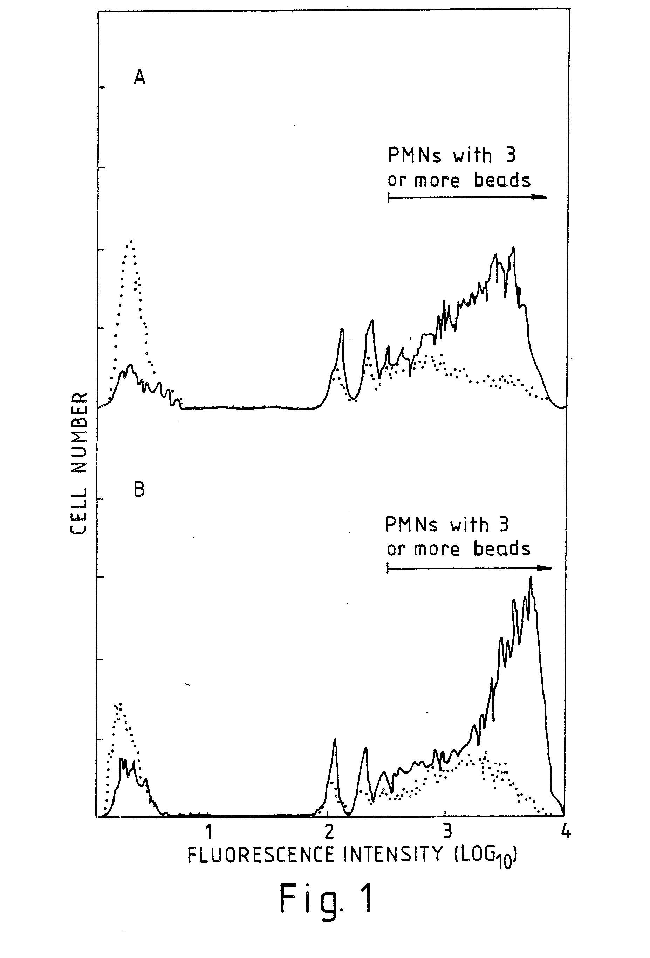

Figure 1. Activation of phagocytosis in PMN by human tumor necrosis factor-alpha from normal (A) and CGD (B) donors. Phagocytosis assay was performed as described in Example 1, General Materials and Methods. Phagocytic cell distribution is illustrated by FACS profiles. Curves closer to the X-axis represent background autofluorescence and/or cells with no beads ingested. In (A): Normal untreated PMN (-); normal PMN treated with human tumor necrosis factor-alpha (--); and, in (B): CGD untreated PMN (-); CGD PMN treated with human tumor necrosis factor-alpha (--).

Phagocytes including polymorphonuclear neutrophils (PMN), monocytes, macrophages and eosinophils have a role in eliminating infections arising from bacterial and fungal pathogens. The participation of phagocytic cells in host defense mechanisms is well established. PMNs can exert antibody-dependent cellular cytotoxic activities against tumor cells and virus-infected cells. Dellegri, F. et al., Immunology 48, 273 (1983). PMNs also demonstrate an unusual process in eliminating pathogens. PMNs first engulf the microbe and then kill the microbe by rapidly consuming a large amount of oxygen. The oxygen consumption is required to produce optimum conditions for the killing of most common bacterial and fungal pathogens. Oxygen-derived metabolic products have been implicated in microbial killing. Babior, B.M., N. Engl. J. Med. 298, 659 (1978).

In certain diseases phagocytes are able to engulf pathogens from blood and tissues but are unable to destroy the phagocytized microorganisms. Infections such as lymphadenitis, pneumonia, liver abscesses and cellulitis arise in such cases of defective phagocytes. Various organisms have been cultured from lesions in such conditions including Staphylococcus aureus, Enterobacter aerogenes, E. coli, Serattia marcescens, Candida albicans and Aspergillus fumigatus. Pathogens engulfed by phagocytes that are unable to kill those pathogens or by defective phagocytes are protected from antibiotics. Thus, antibiotics are of limited use in such patients. This invention encompasses the treatment of infections involving intracellular microorganisms. More particularly the invention encompasses the treatment of infected patients who have normal phagocytes. The invention is particularly applicable to those infections arising in patients with defective phagocytes including those afflicted with chronic granulomatous disease.

Tumor necrosis factor-alpha and its methods of preparation including recovery in recombinant cell culture, are well known, and are disclosed, for example in EP-A-168214. Included within the scope of tumor necrosis factor-alpha are tumor necrosis factor-alpha from recombinant, natural or synthetic resources. Also included are tumor necrosis factor-alpha variants having amino acid substitutions and/or deletions and/or additions, organic and inorganic salts and covalently modified derivatives of tumor necrosis factor-alpha.

For use in the invention herein, tumor necrosis factor-alpha may be formulated into either an injectable or topical preparation. Parenteral fomulations are known and are suitable for use in the invention, preferably for i.m. or i.v. administration. These formulations contain therapeutically effective amounts of tumor necrosis factor-alpha, are either sterile liquid solutions, liquid suspensions or lyophilized versions and optionally contain stabilizers or excipients. Typically, lyophilized compositions are reconstituted with suitable diluents (e.g.) water for injection, saline and the like at a level of about from .001 mg/cc to 10 mg/cc where the biological activity is about $\geq 4 \times 10^7$ units/mg as measured on fibroblast cells (L-M).

Tumor necrosis factor-alpha is formulated into topical preparations for local therapy by including a therapeutically effective concentration of tumor necrosis factor-alpha in a dermatological vehicle. The amount of tumor necrosis factor-alpha to be administered, and the tumor necrosis factor-alpha concentration in the topical formulations, will depend upon the vehicle selected, the clinical condition of the patient, the tumor necrosis factor-alpha species used and the stability of tumor necrosis factor in the formulation. Thus, the therapist will necessarily employ the appropriate preparation containing the appropriate concentration of tumor necrosis factor-alpha in the formulation, as well as the amount of formulation administered depending upon clinical experience with the patient in question or with similar patients. Typically, the concentration for topical formulations is in the range of greater than about from .01 mg/ml to 100 mg/ml. Solid dispersions of tumor necrosis factor-alpha as well as solubilized preparations can be used. Thus the precise concentration to be used in the vehicle will be subject to modest experimental manipulation in order to optimize the therapeutic response. Greater than about 10 mg tumor necrosis factor-alpha/100 grams of vehicle may be useful with 1% w/w hydrogel vehicles in the treatment of infections of the skin. Suitable vehicles, in addition to gels, are oil-in-water or water-in-oil emulsions using mineral oils, petrolatum and the like.

Tumor necrosis factor-alpha optionally is administered topically by the use of a transdermal therapeutic system (Barry, 1983, Dermatological Formulations, p. 181 and literature cited therein). While such systems have been designed largely for transdermal administration of low molecular weight drugs, by definition they are capable of percutaneous delivery. They may be readily adapted to administration of tumor necrosis factor-alpha and associated therapeutic proteins by appropriate selection of the rate-controlling microporous membrane. However, it is not necessary for the skin-binding adhesive to contain a priming dose of tumor necrosis factor-alpha.

Topical preparations of tumor necrosis factor-alpha either for systemic or local delivery may be employed and may contain excipients as described above for parenteral administration and other excipients used in a topical preparation such as cosolvents, surfactants, oils humectants, emollients, preservatives, stabilizers and antioxidants. Any pharmacologically acceptable buffer may be used e.g. tris or phosphate buffers. The topical formulations may also optionally include one or more agents variously termed enhancers, surfactants, accelerants, absorption promoters or penetration enhancers such as, an agent for enhancing percutaneous penetration of the tumor necrosis factor-alpha or other agents. Such agents should desirably possess some or all of the following features as would be known to the ordinarily skilled artisan: be pharmacologically inert, non-promotive of body fluid or electrolyte loss, compatible with tumor necrosis factor-alpha (non-inactivating), and capable of formulation into creams, gels or other topical delivery systems as desired.

In one preferred embodiment a cream base topical preparation comprises:

| Cream Base | grams/100 grams |
|---|---|
| rTNF | 0.01 - 1.0 |
| Glyceryl stearate | 3.0 |
| Isopropyl myristate | 4.0 |
| Propylene glycol | 3.0 |
| PEG-40 stearate | 1.0 |
| Cetyl alcohol | 2.0 |
| Hydroxyethyl cellulose | 0.5 - 1.0 |
| Preservative | 95 |
| Water | 100 |

In yet another example a cream base topical preparation comprises:

| Cream Base | grams/100 grams |
|---|---|
| r-TNF | 0.01 - 1.0 |
| Stearic acid | 15.0 |
| Glycerin | 8.0 |
| Potassium hydroxide | 0.7 |
| Preservative | 95 |
| Water | 76.3 |

In still other examples of a topical preparation a gel or ointment respectively comprises:

| Gel | | grams/100 grams |
| --- | --- | --- |
| rTNF | | 0.01 - 1.0 |
| Sodium phosphate buffer | | 95 |
| Sodium chloride | | 1 |
| Preservative | | 95 |
| Methylcellulose | | 2 |
| Water | | 100 |
| **Ointment** | | |
| r-TNF powdered | | 0.01 - 1.0 |
| Lanolin | | 15 |
| Preservative | | 95 |
| White petrolatum | 95 | 100 |

Tumor necrosis factor-alpha may be administered systemically, rather than topically, by injection i.m. at a dosage of greater than about 25 $\mu$g/m²/day. The dose will be dependent upon the properties of the tumor necrosis factor-alpha species employed, e.g. its activity and biological half life, the concentration of tumor necrosis factor-alpha in the formulation, the site and rate of dosage, the clinical tolerance of the patient involved, the infections afflicting the patient and the like as is well within the skill of the physician.

The tumor necrosis factor-alpha of the present invention may be administered in solution. The pH of the solution should be in the range of pH 5 to 9.5, preferably pH 6.5 to 7.5. The tumor necrosis factor-alpha should be in a solution having a suitable pharmaceutically acceptable buffer such as phosphate, tris (hydroxymethyl) aminomethane-HC1 or citrate and the like. Buffer concentrations should be in the range of 1 to 100 mM. The solution of tumor necrosis factor-alpha may also contain a salt, such as sodium chloride or potassium chloride in concentration of 50 to 750 mM. An effective amount of a stabilizing agent such as an albumin, a globulin, a gelatin, a protamine or a salt of protamine may also be included and may be added to a solution containing tumor necrosis factor-alpha or to the composition from which the solution is prepared.

Systemic administration of tumor necrosis factor-alpha is made daily, generally by intramuscular injection , although i.v. infusion is acceptable. Administration may also be intranasal or by other nonparenteral routes. Tumor necrosis factor-alpha may also be administered via microspheres, liposomes or other microparticulate delivery systems placed in certain tissues including blood. Topical preparations are applied daily directly to the skin or mucosa and then preferably occluded, i.e. protected by overlaying a bandage, polyolefin film or other barrier impermeable to the topical preparation.

Example 1

General Materials and Methods

Recombinant tumor necrosis factor-alpha (rHuTNF-$\alpha$) was cloned and expressed in E. coli and purifie to greater than 99 percent purity. Pennica, D. et al., Nature 312, 724 (1984). Titers of tumor necrosis factor-alpha were calculated by measuring cytolytic activity on actinomycin D treated L-M mouse fibroblast cells. Id. Based on assay results, the specific activity was 4.0 x 10⁷ U/mg protein for recombinant human tumor necrosis factor-alpha. All tumor necrosis factor-alpha preparations were negative for lipopolysaccharide contamination as tested by the Limulus amoebocyte lysate method (Mallinkrodt, St. Louis, MO.).

Peripheral blood monocuclear cells (PBMC) and polymorphonuclear neutrophils (PMNs) were separated as described by Shalaby, M.R. et al., J. Immunol. 135 , 2069, 1985. Briefly PBMC were obtained by centrifugation of heparinized blood over a Ficoll-hypaque gradient. Red blood cells were eliminated from the PMN rich pellet by pyrogen free dextran (Pharmacia Laboratories, Piscataway, N.J.) sedimentation followed by hypotonic lysis in distilled water for approximately 20 seconds. The final PMN cell suspension was routinely of >97 percent pure as monitored in Giemsa staine cytopreparations. Cell viability as determined by trypan blue exclusion was consistently >95 percent.

PBMC were washed three times in Eagles minimal essential medium (MEM, Grand Island Biological Col, Grand Island, N.Y.) supplemented with 10 percent heat inactivated (30 min. at 50°C) fetal bovine serum (FBS, Irvine Scientific, Irvine, CA), 1 percent L-glutamine, 1 percent non-essential amino acids, and penicillin/streptomycin (complete MEM). 4 x 10$^6$ PBMC in 2 ml of complete MEM, were placed in 24 well tissue culture plates (Costar, Cambridge, MA) and stimulated with 5 ng/ml 12-$\beta$-phorbol-12-$\beta$-myristate-13-$\alpha$-acetate (PMA), (Sigma), and/or 1:500 dilution of phytohemagglutin-P (PHA-P), (Difco Laboratories, Detroit, MI). Samples were collected 24, 48, and 72 h after stimulation for tumor necrosis factor-alpha determinations.

Tumor necrosis factor-alpha levels were determined by specific ELISA assays. The ELISA for tumor necrosis factor-alpha detects $\geq$ 0.4 ng/ml. Vadhan-Raj, S., et al., J. Clinical Oncology 4, 137 (1986).

PMNs were suspended (2.5 x 10$^6$/ml) in minimal essential medium (GIBCO) supplemented with 5 percent heat-inactivated FBS (Irvine Scientific). Aliquots of 0.5 ml cells were dispenses in 12 x 75 mm plastic tubes in the absence or presence of varying human tumor necrosis factor-alpha doses and incubated on an orbital shaker at 37°C for 20 minutes. Sixy microliters of a monodispersed suspension of 1.5 micron fluorescenated latex beads (10$^9$/ml) (Polysciences Inc., Warrington, PA) were added to each culture and the tubes were further incubated for 60 minutes at 37°C. Cells were fixed by the addition of an equal volume of 2 percent glutaraldehyde (Sigma, St. Louis, MO), and centrifuged over FBS gradients to eliminate uningested beads. The number of phagocytic PMNs was determined using a fluorescence activated cell sorter (FACS IV, Becton Dickinson and Co., Sunnyvale, CA).

PMNs (8 x 10$^{4-}$) were analyzed by FACS IV for forward-angle light scatter (cell size) and fluorescence (phagocytized latex beads) as they flowed past an Argon laser. These measurements were performed on a single cell basis and displayed as frequency distribution histograms. The FACS IV was programmed to electronically discriminate between uningested beads, cell debris, cell clumps, and cells containing fluorescent beads. PMNs containing ingested beads were quantitated on the basis of their relative fluorescence displayed on the histograms. Some samples were also subjected to microscopic examination to confirm the elimination of uningested or attached beads.

Analysis of antibody dependent cellular cytotoxic (ADCC) activities was done by suspending PMNs in complete media and incubating for 2 hr at 37°C in the absence or presence of human tumor necrosis factor-alpha at various doses. Thereafter, triplicate volumes of 0.1 ml containing varying numbers of PMN were transferred to round-bottom microtiter plates (Costar, Cambridge, MA). Aliquots of 0.1 ml/well containing 10$^4$ $^{51}$Cr-labeled chicken red blood cels (CRBC) and antibody were then added. After 4 hr of incubation, the supernatants were harvested (Skatron, Inc., Sterling, VA) and the radioactivity was determined by using an automatic gamma counter model 28037 (Micromedic Systems, Inc., Horsham, PA). Specific target lysis, expressed as percent cytotoxicity, was calculated as follows:

$$\text{Percent cytotoxicity} = \frac{A - B}{C - B} \times 100$$

where A represents mean cpm in test supernatants (targets co-cultured with control or treated PMN), B represents mean cpm spontaneous release (targets cultured alone), and C represents mean cpm maximum release (targets lysed with 1% Nonidet P-40). Specific cytotoxicity tested routinely in the absence of antibody was 5% or less.

Example 2

Enhancement of PMN-ADCC

. Results from at least 20 donors tested showed that the treatment of PMN with human tumor necrosis factor-alpha caused an enhancement in over 90 percent of the cases. Results in Figure 1 shown that human tumor necrosis factor-alpha resulted in enhancement of PMN-ADCC activity. Studies of dose-response analyses were conducted and repesentative results from three experiments are summarized in Table I. Consistently, a substantial enhancement of PMN-ADCC was evident after treatment with all of the doses tested for human tumor necrosis factor-alpha. pc 0.05 for treatment with 1 or 10 U/ml.

### Table 1

### Effect of Different Doses of
### Human Tumor Necrosis Factor-Alpha
### on PMN-mediated ADCC against CRBC

| | Percent Cytotoxicity After Treatment with Human Tumor Necrosis Factor-(U/ml)[a] | | | |
|---|---|---|---|---|
| E:T[b] | 0.0 | 0.1 | 1.0 | 10.0 |
| 8:1 | 26 | 37 | 42 | 46 |
| 4:1 | 18 | 25 | 34 | 39 |
| 2:1 | 11 | 14 | 22 | 27 |

[a] Cells were treated with human tumor necrosis factor-alpha. After 2 hrs. of incubation of cells, 0.1 ml containing varying numbers of untreated or human tumor necrosis factor-alpha treated PMN were transferred to microtiter plates. $^{51}$Cr-labeled CRBC and antibody were added, and after 4 hrs. of incubation the supernatants were harvested and their radioactivity was determined. Specific target lysis of triplicate cultures ($^{51}$Cr release) is expressed as percent cytotoxicity. In each case, elimination of antibodies against CRBC resulted in 0-5 percent cytotoxicity.

[b] E:T = effector to target ratio.

### Example 3

Enhancement of $O_2^-$ Production

As shown in Table 2, after 12-$\beta$-phorbol-12-$\beta$-myristate-13-$\alpha$ acetate (PMA) stimulation PMNs from normal donors produced significant levels of $O_2^-$ (0.44 nm for unstimulated compared to 2.59 from PMA stimulated; p<0.001). PMA is known to effect PMN activity. $O_2^-$ levels ranged from 0.57 nm $O_2^-$ to 5.6 nm $O_2^-$ for PMA stimulated and from 0 to 1.37 nm $O_2^-$ for media controls. In contrast, PMN from the three male X-linked CGD donors (DD, JM, and RD) produced significantly lower levels of $O_2^-$ after PMA stimulation compared to normals (0.17 nm $O_2^-$ versus 2.59 nm $O_2^-$, respectively) (p<0.001). $O_2^-$ levels produced by the two female CGD carriers (JR and CM) were not significantly different from normals. The results confirmed the clinical diagnosis of X-linked CGD in the three male patients.

### Table 2

$O_2^-$ Production by PMN Derived from Normal and CGD Donors

| Donors | nm $O_2^-$ Production after Stimulation with[a] | |
| --- | --- | --- |
| | Media | PMA |
| Normals[b] | 0.44 ± 0.50 | 2.59 ± 1.54 |
| CGDs[c] | 0 ± 0.01 | 0.17 ± 0.24 |
| CGD Carriers[d] | 0.12 ± 0.10 | 1.6 ± 1.1 |

[a] $O_2^-$ measurements were determined between 50 and 90 minutes for normals and 90 to 120 minutes for CGDs and CGD carriers.

[b] Mean results of twenty tests from 15 normal donors.

[c] Mean results of four tests from three male X-linked CGD donors.

[d] Mean results of six tests from two CGD-carrier donors.

As shown in Table 3, human tumor necrosis factor-alpha induced significant levels of $O_2^-$ 60 minutes after stimulation in PMNs obtained from peripheral blood of normal donors compared to untreated controls ($p < 0.001$). While both human tumor necrosis factor-alpha and tumor necrosis factor-beta (also called lymphotoxin) stimulate neutrophil activity, human tumor necrosis factor-alpha showed greater enhancement of neutrophil activity than tumor necrosis factor-beta.

### Table 3

$O_2^-$ Production by rHuTNF-α

| Stimulus | nm $O_2^-$ Production[a] |
| --- | --- |
| Media | 0.06 |
| PMA | 3.90 |
| PMA + SOD | 0.19 |
| $2.5 \times 10^3$ U rHuTNF-α | 2.50 |

[a] Determined 60 minutes after stimulation.

As shown in Table 4, human tumor necrosis factor-alpha stimulated PMNs obtained from normal, CGD carriers and X-linked CGD donors to produce significantly higher levels of $O_2^-$ compared to the unstimulated controls. Both PMA and human tumor necrosis factor-alpha induced $O_2^-$ was inhibited by superoxide dismutase (SOD), thereby verifying that the activity measured was in fact $O_2^-$ and that PMN from X-linked CGD donors can produce $O_2^-$.

### Table 4

### $O_2^-$ Production after Stimulation with rHuTNF-α

| Stimulus | nm $O_2^-$ Produced[a] | |
| --- | --- | --- |
| | X-Linked-CGD[b] | CGD Carrier[c] |
| Media | 0 | $0.07 \pm 0.09$ |
| PMA | $0.29 \pm 0.27$ | $1.74 \pm 0.23$ |
| PMA + SOD | $0.05 \pm 0.08$ | $0.10 \pm 0.13$ |
| $1 \times 10^4$ U rHuTNF-α | $0.67 \pm 0.28$ | $1.09 \pm 0.17$ |
| $2.5-5 \times 10^3$ U rHuTNF-α | $0.48 \pm 0.08$ | $0.85 \pm 0.08$ |
| $1 \times 10^4$ U rHuTNF-α + SOD | $0.03 \pm 0.04$ | $0.20 \pm 0.10$ |

[a] $O_2^-$ was determined between 60 and 90 minutes after stimulation. Results are mean of three to four replicates.

[b] Mean of 3 tests from 3 donors.

[c] Mean 2 tests from 2 donors.

Example 4

Stimulation of Phagocytosis

The augmentation of PMN mediated phagocytosis by human tumor necrosis factor-alpha stimulation was studied. As shown in Figure 1A and 1B panels, PMNs from both normal (MW) and CGD (JM) donors, respectively, showed enhanced phagocytosis after stimulation with 10 U/ml human tumor necrosis factor-alpha as determined by the number of PMNs with greater than three phagocytized latex beads. The results confirm that PMNs from CGD donors retain normal phagocytosis despite the $O_2^-$ generating system defect.

To determine whether the immune dysfunction in CGD donors was confined to the $O_2^-$ generating system in the PMn or might also effect cytokine production, the ability of peripheral blood mononuclear cells to produce IFN-γ and TNF-α after stimulation with PHA-P, PMA or PHA-P/PMA was studied. Results from one of three experiments are shown in Table 5.

## Table 5

### Production of Interferon-gamma and Tumor Necrosis Factor-alpha by Peripheral Blood Mononuclear Cells of Normal CGD Carrier and CGD Donors

| Donor | Stimulus[a] | Lymphokine Production | |
|---|---|---|---|
| | | IFN-$\gamma$ (ng/ml) | TNF-$\alpha$(pg/ml) |
| TR (normal) | - | 1.14 | LTS[b] |
| | PHA-P | 22.75 | 787.95 |
| | PMA | 1.95 | 783.4 |
| | PHA-P/PMA | 84.34 | 8310.25 |
| JR (carrier) | | 1.06 | 220.3 |
| | PHA-P | 12.26 | 1106.3 |
| | PMA | 1.74 | 2236.55 |
| | PHA-P/PMA | 62.02 | 15028.0 |
| DD (CGD) | - | 1.25 | 329.4 |
| | PHA-P | 102.20 | 3306.45 |
| | PMA | 535.65 | 7341.0 |
| | PHA-P/PMA | 702.15 | 15940.0 |
| JM (CGD) | - | 1.49 | 249.7 |
| | PHA-P | 17.92 | 815.7 |
| | PMA | 187.00 | 4563.9 |
| | PHA-P/PMA | 446.55 | 7289.0 |

[a] Phytohemagglutinin-P (PHA-P) was used at a final concentration of 1:500 and 12-B phorbol 12-B myristate 13-$\alpha$-acetate (PMA) at 10 ng/ml

[b] Lower than standard (LTS)

Only nanogram levels of interferon-gamma were produced after stimulation of PBMC from normal and CGD-carriers with PMA. However, surprisingly, PMA alone stimulated the production of high levels of IFN-$\gamma$ in PBMC from the two X-linked CGD patients (DD and JM) (1.95 ± 0.2 ng/ml for normal and CGD carrier compared to 361 ± 247 ng/ml for X-linked CGD donors on day 3, p<0.001). In addition, PBMC from X-linked CGD donors stimulated with PHA-P/PMA produced significantly greater interferon-gamma compared to normal or CGD carrier on day 3 (574 ± 181 ng/ml vs 73 ± 16 ng/ml, p<0.001) and days 1 and 2 (data not shown). In additional studies (Table 5) similar increases in tumor necrosis factor-alpha production were also obtained, i.e., PBMC from X-linked CGD donors stimulated with PMA produced significantly greater levels of tumor necrosis factor-alpha production compared to normal or CGD-carriers on day 3 (5952 ± 1389 pg/ml vs 1510 ± 727 pg/ml), p<0.001) and days 1 and 2 (data not shown). In contrast, PBMC from CGD, normal or CGD carrier donors stimulated with PHA-P/PMA produced similar levels of tumor necrosis factor-alpha on day 3 (11669 ± 4750 pg/ml for normal CGD carrier donors vs 11615 ± 6117 pg/ml for X-linked CGDs, p - ns) and day 2 (data not shown). Tumor necrosis factor-alpha production after PHA-P/PMA stimulation in the X-linked CGD group was significantly higher only on day 1 (data not shown).

These results confirm that PBMC from CGD donors compared to normal donors show variable alterations in their ability to produce IFN-$\gamma$ after mitogenic stimulation. Similar alterations were observed in tumor necrosis factor-alpha production. PBMC from CGD donors also produced significantly higher concentrations of both TNF-$\alpha$ and IFN-$\gamma$ after PMA and PMA/PHA-P stimulation compared to PBMC from normal donors. Previous studies have shown that PMA functions in PBMC cultures from normal donors as a co-mitogen which alone fails to stimulate significant levels of IFN-$\gamma$ or interleukin-2 production but significantly enhances lymphokine production in the presence of a mitogen. Pearlstein, K.T., et al., J. Biol.

Response Modifiers $\underline{2}$, 81-91 (1983). These results indicate therefore that significant differences exist in the signals required for activation of PBMC in CGD patients compared to PBMC from normal donors and suggest that the immune dysregula tion in this disease is of a greater magnitude than previously thought. Segal, A.W., The Lancet, June 15, 1378-1382, (1985).

Example $\underline{5}$

Tumor Necrosis Factor-alpha Effect on Neutrophil Activation by <u>Candida</u> <u>albicans</u>

The effect of recombinant tumor necrosis factor-alpha on PMN activation by <u>Candida</u> <u>albicans</u> was studied. Previous data suggested differences in PMN responses elicited by opsonized particles, (zymosan or hyphae) compared with unopsonized hyphae. PMNs was obtained and separated from peripheral blood. PMNs were incubated with tumor necrosis factor-alpha (100 units $10^6$ PMNs) for thirty (30) minutes and no effect was observed on unstimulated baseline release of superoxide ($O_2^-$). Preincubation of PMNs with tumor necrosis factor-alpha (100 units/$10_6$) for thirty minutes augmented $O_2^-$ generation by zymosan (20.2%) as well as by both opsonized (38.6%) and unopsonized hyphae (42.0%). Pertussis toxin (PT), a known inhibitor of superoxide ($O_2^-$) release, 500 ng/ml for 2 hours, and calcium (Ca + +) chelation (intracellular, by loading with an organic buffer, BAPTA; and, extracellular, with EGTA). BAPTA or EGTA, with or without PT, almost completely ablate (95-99%) $O_2^-$ responses of PMN to opsonized zymosan, irrespective of tumor necrosis factor-alpha pretreatment. However, there was only a partial reduction in respiratory burst responses elicited from tumor necrosis factor-alpha stimulated PMN by hyphae, whether opsonized or not (respective inhibition with opsonized vs. unopsonized hyphae: $24.6^+4.3$ vs $25.0 + 2.4\%$ by PT, $20.0 + 8.4$ vs $45.5 + 4.9\%$ by BAPTA, $47.3 + 2.4$ vs $27.5 + 1.4\%$ by EGTA, and $68.9 + 2.8$ vs $54.3 + 3.3\%$ by all three in combination). In all cases, i.e. for all sets of conditions, PMN $O_2^-$ responses to hyphae were augmented significantly by tumor necrosis factor-alpha ($p<0.05$). These data suggest that tumor necrosis factor-alpha acts to augment the PMN respiratory burst at a site in the activation process proximal to involvement of PT-inhibitable guanine nucleotide regulatory proteins or "second messenger" responses mediated by detectable calcium ion fluxes with changes in cytosolic calcium concentrations.

**Claims**

1. Use of tumor necrosis factor-alpha in the manufacture of a medicament for treatment of an infectious disease.

2. Use according to claim 1 wherein the infectious disease is associated with a defect in phagocytic cells.

3. Use according to claim 1 or claim 2 wherein the infec-tious disease is associated with a defect in polymorpho-nuclear neutrophils.

4. Use according to claim 3 wherein the infectious disease is consequent to chronic granulomatous disease.

5. Use according to any preceding claim wherein the medicament is for intramuscular administration.

6. Use according to any one of claims 1 to 4 wherein the medicament is for intravenous administration.

7. Use according to any one of claims 1 to 4 wherein the medicament is for topical administration.

8. Use according to any one of the preceding claims wherein the medicament further includes an antibiotic for simultaneous or sequential administration.

Fig. 1